Europäisches Patentamt

European Patent Office

Office européen des brevets

(19)

(11) Veröffentlichungsnummer: **0 241 804 B1**

(12) **EUROPÄISCHE PATENTSCHRIFT**

(45) Veröffentlichungstag der Patentschrift: **15.01.92**

(51) Int. Cl.5: **A61K 33/10**, A61K 47/00

(21) Anmeldenummer: **87104752.8**

(22) Anmeldetag: **31.03.87**

(54) **Konservierte Antacida-Zubereitungen.**

(30) Priorität: **10.04.86 DE 3612086**

(43) Veröffentlichungstag der Anmeldung:
**21.10.87 Patentblatt 87/43**

(45) Bekanntmachung des Hinweises auf die
Patenterteilung:
**15.01.92 Patentblatt 92/03**

(84) Benannte Vertragsstaaten:
**AT BE CH DE ES FR GB IT LI NL**

(56) Entgegenhaltungen:
WO-A-84/04886          WO-A-85/04806
CH-A- 633 187          DE-A- 2 631 947
US-A- 3 577 532        US-A- 4 409 202

JOURNAL OF THE AMERICAN PHARMACEU-
TICAL ASSOCIATION, "Scientific edition",
Band 47, Nr. 4, April 1958, Seiten 294-296,
American Pharmaceutical Association, Easton, Pa, US; J.C. MARUZZELLA et al.: "The in
vitro antibacterial activity of essential oils
and oil combinations"

(73) Patentinhaber: **BAYER AG**

**W-5090 Leverkusen 1 Bayerwerk(DE)**

(72) Erfinder: **Fritsch, Christian, Dr.
Schlinghofenerstrasse 33
W-5068 Odenthal(DE)**
Erfinder: **Wetzstein, Heinz-Georg, Dr.
Schleiermacherstrasse 13
W-5090 Leverkusen 1(DE)**

**Beschreibung**

Die vorliegende Erfindung betrifft neue, haltbare Antacida-Zubereitungen, die Zimtöl als alleiniges Konservierungsmittel enthalten.

Es ist bekannt, daß bei der Herstellung oder insbesondere beim Gebrauch Von Antacida-Suspensionen Mikrooraganismen eingeschleppt werden können. In den wässrigen Zubereitungen können sich diese Mikroorganismen gut vermehren, die Zubereitung durch Gärungsprozesse gefährden und durch ihr pathogenes Potential eine ernstzunehmende Gefahr darstellen.

Es hat deshalb nicht an Versuchen gefehlt, solche Antacida-Zubereitungen haltbar zu machen. Vorgeschlagen wurde sowohl der Zusatz von Haltbarmachern als auch physikalische oder chemische Verfahren zur Sterilisierung von wässrigen Zubereitungen. Den physikalischen oder chemischen Sterilisierungsverfahren haftet der Nachteil an, daß sie die wässrigen Suspensionen nicht vor Einschleppen von Mikroorganismen beim Gebrauch oder Verbrauch schützen können.

Aus der DE-OS 2 631 947 ist bereits bekannt geworden, Arzneimittelzubereitungen mit einer Kombination aus Ethanol und ätherischen Ölen zu konservieren.

Aus der PCT-Anmeldung WO 84/04886 sind Zubereitungen bekannt, welche mit Zimtaldehyd und Parabenen konserviert werden.

Es wurde überraschend gefunden, daß Antacida-Zubereitungen durch den Zusatz von Zimtöl alleine wirksam gegen mikrobiellen Verderb geschützt werden können.

Gegenstand der vorliegenden Erfindung sind deshalb Antacida-Zubereitungen, die als alleiniges Konservierungsmittel Zimtöl enthalten.

Ein weiterer Gegenstand der Erfindung ist die Verwendung von Zimtöl zur Konservierung von Antacida-Zubereitungen sowie ein Verfahren zur Herstellung von konservierten Antacida-Zubereitungen, welches dadurch gekennzeichnet ist, daß man den Antacida neben üblichen Hilfs- und Zusatzstoffen als Konservierungsmittel Zimtöl zugibt und die Mischung anschließend homogenisiert. Eine solche Homogenisierung kann in den üblichen Vorrichtungen erfolgen.

Unter Antacida-Zubereitungen im Sinne der vorliegenden Erfindung werden Gele, Suspensionen, Aufschlämmungen sowie Pasten verstanden, welche säurebindend und/oder säureneutralisierend wirken.

Unter Antacida werden Verbindungen oder solche Verbindungen enthaltende Zubereitungen verstanden, die den pH-Wert des Mageninhalts durch teilweise oder vollständige Neutralisation der Magensäure (Salzsäure) anheben. Antacida reduzieren nicht die Menge an Salzsäure, welche von der Schleimhaut des Magens sezerniert wird, sondern neutralisieren lediglich bereits sezernierte Salzsäure.

Suspensionen im Sinne der vorliegenden Erfindung sind disperse Systeme, deren innere (disperese) Phase aus Feststoffpartikeln und deren äußere Phase (Dispersionsmittel, Vehikel ) aus einer Flüssigkeit bestehen. Zu der Form Suspension werden nur solche Präparate gezählt, die sich durch eine zusammenhängende äußere Phase auszeichnen und im allgemeinen ohne zusätzliche Schubspannung, allein unter dem Einfluß der Schwerekraft von selbst zu fließen beginnen. Der Feststoffanteil einer derartig definierten Suspension liegt zwischen 0,5 und 40 Gewichtsprozent.

Pasten im Sinne der vorliegenden Erfindung sind Salben mit einem hohen Anteil an puderförmigen Feststoffen und deshalb hoher Konsistenz. Salben sind Gele von plastischer Verformbarkeit, die Arzneistoffe gelöst, emulgiert oder suspendiert enthalten. Der Feststoffanteil in Pasten beträgt ca. 15-70 Gew.%.

Gele im Sinne der vorliegenden Erfindung sind disperse Systeme "fest/flüssige", bei denen im Gegensatz zu den Solen und Suspensionen die disperse Phase nicht mehr frei beweglich ist. Sie weist im Gelzustand keine Partikel im Sinne selbständiger kinetischer Eigenheiten mehr auf, sondern ist aufgrund besonderer struktureller Anordnung räumlich fixiert. Das System ist demnach bikohärent. Besteht die disperse Phase eines Gels aus kleinen, meist anorganischen Partikeln wie Betonit, Aluminiumhydroxid u.a., so wird häufig der Name Magma gebraucht.

Vorzugsweise weisen die Antacida-Zubereitungen einen pH-Wert größer als 7, besonders bevorzugt 7,1 bis 9,5 auf.

Erfindungsgemäß wird Zimtöl als alleiniges Konservierungsmittel eingesetzt. Vorzugsweise wird das erfindungsgemäße Konservierungsmittel für Antacida-Zubereitungen in Mengen von 0,01 bis 0,1 Gew.-%, besonders bevorzugt von 0,02 bis 0,07 Gew.-% eingesetzt.

Besonders bevorzugt wird als Antacidum Hydrotalcit, ein Aluminium-Magnesiun-Hydroxid-Carbonat der allgemeinen Formel $Al_2O_3 \cdot 6\, MgO \cdot CO_2 \cdot 12\, H_2O$ (US-PS 3 539 306) in Mengen von 1 bis 70 Gew.%, besonders bevorzugt 5-30 Gew.% eingesetzt.

Eine typische und erfindungsgemäß besonders bevorzugte Zusammensetzung wird nachstehend angegeben:

2

```
Hydrotalcit  1 - 70    ;   5 - 30   ,   10 - 20

Zimtöl    0,01 - 0,1  ;0,02 - 0,08 , 0,03 - 0,06

Verdik-   0,1 - 10   ; 0,7 - 5   ,  0,8 - 3

kungsmittel

Rest: Wasser und ggf. Hilfsmittel.
```

Alle Angaben sind in Gewichtsprozent.

Unter Hilfsmitteln im Sinne der vorliegenden Erfindung werden Geschmacksstoffe, Süßungsmittel , Viskositätsbildner bzw. Gelbildner, Verdickungsmittel sowie weitere pharmakologisch unbedenkliche Zuschlagstoffe verstanden.

Die Viskositäts- bzw. Gelbildner werden in Mengen von 0,1 bis 10 Gew.%, vorzugsweise 0,7 bis 5 Gew.%, besonders bevorzugt 0,8 bis 3 Gew.% eingesetzt. Vorzugsweise werden hierunter verstanden: Cellulosederivate, Polyvinylalkohol, Polyacrylsäurederivate, Alginsäure und (-Salze), Pektin, Traganth, Gummi Arabicum, Bentonit sowie hochdisperse Kieselsäure.

Die erfindungsgemäßen Zubereitungen enthalten noch Wasser sowie bei Bedarf ggf. noch weitere Hilfsstoffe, z.B. dichteerhöhende Substanzen, wie Saccharose, Fructose, Glucose, Mannitol, Sorbitol, Xylitol oder Glycerol;

Süßungsmittel wie z.B. obengenannte dichteerhöhende Substanzen, Saccharin-Natrium, Cyclamat oder Aspartame;

Aromen wie z.B. Aromen von Pfefferminz, Zitrone, Vanille, Erdbeere, Stachelbeere, Kirsche, Banane, Pfirsich oder Aprikose;

Netzmittel wie z.B. Natriumlaurylsulfat, Pluronic, Polyoxyethyliertes Rizinusöl;

pH-Wert und Ionenkonzentration beeinflussende Substanzen wie z.B. Zitronensäure oder Natriumcitrat, Natriumacetat oder Natriumchlorid.

Gegebenenfalls können noch weitere Wirkstoffe zugesetzt werden, als solche kommen in Betracht:

Adsorbentien wie z.B. Attapulgit, med. Kohle, Kaolin;

Antiflatulentien, z.B. Simethicon;

Lokalanesthetika, z.B. Oxethazaine;

Antiulcera, z.B. Sucralfat oder Carbenoxolon;

Anticholinergika, z. B. Propanthelin;

pflanzliche Bestandteile oder Auszüge wie z.B. Kamille, Fenchel, Anis, Kümmel, Thymian, Süßholz, Angelika, Clamus oder Rharbarber.

Die erfindungsgemäßen, konservierten Antacida-Zubereitungen können auch in halbfester, sogenannter pastöser Form vorliegen. Der Antacidum-Anteil ist in solchen Zubereitungen relativ hoch, er beträgt etwa 15 bis 70 Gew.%.

Als weitere säurebindende Wirksubstanzen können in den erfindungsgemäßen Antacida-Zubereitungen die nachstehend aufgeführten Stoffe eingesetzt werden. Diese Aufzählung ist nur beispielhaft gedacht und nicht abschließend. Erfindungsgemäß bevorzugt werden solche säurebindenden Wirksubstanzen eingesetzt, die in der fertigen Zubereitung einen pH-Wert von 7,1 bis 9,5 bewirken:

Aluminiumhydroxid, Naß- oder Trockengele

Aluminium-Natriumcarbonat-dihydroxid

Aluminium-Magnesium-Silikat-hydrat

Aluminium-Glycinat-dihydroxid

Calciumcarbonat

Calciumhydrogenphosphat

Hydrotalcit

Magaldrat

Natriumhydrogencarbonat

Magnesiumhydroxid, Mageniumoxid

Magnesiumcarbonat (basisch)

Magnesiumtrisilikat

Unter dem erfindungsgemäßen Konservierungsmittel Zimtöl wird verstanden:

Zimtöl : Zimtöl DAB 7 ist das ätherische Öl aus der Rinde von Cinnamonum ceylanicum (Blume). Es enthält 64-76 % (G/G) Zimtaldehyd und 5-11 % (V/V) Eugenol und Zimtsäure. Zimtöl NF XVI ist das ätherische Öl aus der Rinde von Cinnamonum cassia (Nees). Es enthält mindestens 80 % Aldehyde.

Erfindungsgemäß können alle Handelsformen des Zimtöls, die pharmakologisch unbedenklich sind, eingesetzt werden.

Es ist ausgesprochen überraschend, daß mit dem erfindungsgemäßen Einsatz der niedrigen Konzentrationen des besagten Konservierungsmittels, die bisher nur unzureichend und schwierig zu konservierenden Antacida-Zubereitungen wirksam und in einfacher Weise vor mikrobiellem Verderb geschützt werden können. Die erfindungsgemäße Konservierung ist über einen langen Zeitraum wirksam.

Die vorliegende Erfindung bietet eine Reihe von Vorteilen. Sie ermöglicht:

a) einen wirksamen Schutz von Antacida-Zubereitungen vor mikrobiellem Verderb, der auch während der Aufbrauchzeit des Arzneimittels bestehen bleibt.

b) eine Behandlung von Personen, die auf die bisher üblichen Konservierungsmittel allergisch reagieren.

c) eine allgemeine Verträglichkeit durch die vergleichsweise geringen Konzentrationen des Konservierungsmittels und

d) eine Vermeidung der Geschmacksbeeinträchtigung, wie sie durch die herkömmlichen Konservierungsmittel verursacht wird.

Der erfindungsgemäße Zusatz von Zimtöl beeinträchtigt den Einsatz der sonst üblichen Formulierungshilfsstoffe in keiner Weise.

Der Nachweis der einwandfreien Haltbarmachung von Antacida-Zubereitungen erfolgte mit Hilfe geeigneter Konservierungsbelastungstests wie sie z.B. in der Britischen Pharmakopöe von 1980 (British Pharmacopoeia 1980, Addendum 1982, Revised Apendix XVIC: Efficacy of Antimicrobial Preservatives in Pharmaceutical Products) oder der Pharmakopöe der Vereinigten Staaten (The United States Pharmacopeia XXI (1985) Antimicrobial Preservatives Effectiveness, S. 1151-1156) beschrieben werden.

Neben einer Richtlinie der WHO für nichtsterile Produkte, die eine Begrenzung des Keimgehalts in nichtsterilen oralen Arzneimitteln auf 1000/ml fordert, gelten unterschiedlich hohe Anforderungen hinsichtlich der Abtötungsrate für bestimmte Testmikroorganismen, je nach Pharmakopöe. Die britische Pharmakopöe verlangt z.B. eine Verminderung der Bakterienkonzentration um 2 Zehnerpotenzen innerhalb von 7 Tagen sowie keine Vermehrung von Hefen und Schimmelpilzen (s. Tab. 1).

Tabelle 2 zeigt die Ergebnisse von Konservierungstests, bei denen Hydrotalcitsuspensionen mit einer Reihe von Konservierungsmitteln in verschiedenen Konzentrationen eingesetzt wurden. Konservierungsmittel, deren Wirkprinzip (soweit bekannt) in erster Linie auf einer Äquilibrierung der cytoplasmatischen Wasserstoffionenkonzentration mit derjenigen der Suspension beruht (Benzoesäure, Sorbinsäure) erwiesen sich - auch in hohen Konzentrationen - als unwirksam; es kam sogar zur Vermehrung von Bakterien und Hefen. Ebenso waren Kombinationen aus Sorbinsäure und Solbrolen (p-Hydroxybenzoesäureester) sowie Chlorhexidin unwirksam. Formulierungen, denen Zimtöl zugesetzt worden war, erwiesen sich als gut konserviert.

Tabelle 1    Anforderungen an die Konservierungsgüte flüssiger oraler Arzneimittel

| Monographie | Reduktion des Keimgehalts in Zehnerpotenzen | |
| --- | --- | --- |
| | Bakterien | Hefen/Schimmelpilze |
| British Pharmacopoeia 1980 (Addendum 1982, Prevised Appendix XVIC) | 2 (7 d) | k |
| Deutsches Arzneibuch 9 | 3 (14 d) | 1 (14 d) |
| Féderation International Pharmaceutique | 2 | k |
| United States Pharmacopeia XXI | 3 (14 d) | k |

d = Tage nach Beimpfung; k = keine Vermehrung

EP 0 241 804 B1

EP 0 241 804 B1

Tabelle 2    Konservierungsgüte von Hydrotalcit Suspension 10 % G/V

| Konservierungsmittel | Konzentration (Gew.%) | pH | Keimzahl nach 7 Tagen | | | Konservierungs- güte** |
|---|---|---|---|---|---|---|
| | | | Bakterien | C.albicans | A.niger | |
| ohne | | 8,8 | ↑ | (↑) | (↑) | - |
| ohne | | 8,8 | ↑ | k | k | - |
| Benzoesäure | 0,2 | 8,3 | ↑ | k | k | - |
| Chlorhexidinacetat | 0,02 | 8,8 | ↑ | k | k | - |
| Chlorhexidinacetat | 0,01 | 8,7 | k | k | k | - |
| Eugenol | 0,1 | 8,7 | ↓>5 | ↓>4 | ↓ | + |
| Sorbinsäure | 0,4 | 8,0 | ↑ | (↑) | k | - |
| Sorbinsäure | 0,2 | 8,3 | ↑ | k | k | - |
| Sorbinsäure + Solbrol P | 0,4 + 0,004 | 7,8 | ↑ | ↓ | k | - |
| Sorbinsäure + Solbrol M | 0,2 + 0,1 | 8,3 | ↑ | k | k | - |
| Sorbinsäure + Solbrol M | 0,2 + 0,05 | 8,4 | (↑) | (↑) | k | - |
| Sorbinsäure + Solbrol M | 0,1 + 0,1 | 8,5 | (↑) | k | k | - |
| Sorbinsäure + Solbrol M | 0,1 + 0,05 | 8,6 | ↑ | (↑) | k | - |

6

**Fortsetzung Tabelle 2**   Konservierungsgüte von Hydrotalcit Suspension 10 % G/V

| Konservierungsmittel | Konzentration (Gew.%) | pH | Keimzahl nach 7 Tagen Bakterien+ | C.albicans | A.niger | Konservierungs-güte ** |
|---|---|---|---|---|---|---|
| Zimtöl / Pfefferminzaroma ++ | 0,1 + 0,1 | 8,5 | ↓>7 | ↓>6 | ↓>6 | + |
| Zimtöl / Pfefferminzaroma ++ | 0,05 + 0,1 | 7,9 | ↓>6 | ↓>6 | ↓>6 | + |
| Zimtöl / Pfefferminzaroma ++ | 0,02 + 0,1 | 8,6 | ↓>7 | ↓>6 | ↓>6 | + |
| Zimtöl / Pfefferminzaroma ++ | 0,01 + 0,1 | 8,7 | ↓>6 | ↓>6 | ↓>6 | -*** |

| | | |
|---|---|---|
| * | = | Keimzahl nach 2 Tagen nur geringfügig vermindert |
| ** | = | Kriterium: Verminderung der Keimzahl um 2 Zehnerpotenzen nach 7 Tagen |
| *** | = | Keimzahlzunahme bis zum 28. Tag |
| ↑↓ | = | Zu- bzw. Abnahme der Keimzahl um etwa 1 Zehnerpotenz |
| (↑) | = | Zunahme der Keimzahl um 1/2 bis 1 Zehnerpotenz |
| ↓>X | = | Abnahme der Keimzahl um X Zehnerpotenzen |
| k | = | Keimzahl konstant |
| + | = | Mischung aus Staphylococcus aureus, Pseudomonas aeruginosa und Escherichia coli |
| ++ | = | Pulverförmiges Pfefferminzaroma mit 20 % Pfefferminzölgehalt |

Die vorliegende Erfindung soll durch die nachfolgenden Beispiele noch näher beschrieben werden:

Beispiel 1

15.5 g Carboxymethylcellulose werden in 60/ml entmineralisiertem Wasser heiß gelöst. Nach dem Abkühlen werden 100 g Hydrotalcit, 5 g disperse Kieselsäure, 0,2 g Saccharin-Natrium, 1 g Pfefferminzaroma und 0,2 g Zimtöl eingerührt. Die so erhaltene Mischung wird mit entmineralisiertem Wasser auf einen Liter aufgefüllt und zuletzt homogenisiert.

Diese Suspension ist gegen mikrobiellen Verderb geschützt und kann als Antacidum appliziert werden.

Beispiel 2

| Hydrotalcit-Paste | |
|---|---|
| Hydrotalcit | 30,0 g |
| hochdisp. Siliciumdioxid | 1,0 g |
| Magnesiumalginat | 1,2 g |
| Glycerin, wasserfrei | 20,0 g |
| Pfefferminzaroma | 0,1 g |
| Zimtmöl DAB7 | 0,02 g |
| Wasser | 47,68 g |
| | 100,0 g |

Diese Paste ist über einen Zeitraum von mehr als 10 Monate stabil.

**Patentansprüche**
**Patentansprüche für folgende Vertragsstaaten : AT, BE, CH, LI, DE, FR, GB, IT, NL**

1. Konservierte Antacida-Zubereitungen, enthaltend Zimtöl als einziges Konservierungsmittel.

2. Konservierte Antacida-Zubereitungen nach Anspruch 1 enthaltend Zimtöl in Mengen von 0,01 bis 0,1 Gew.-%.

3. Konservierte Antacida-Zubereitungen nach Anspruch 1 enthaltend Zimtöl in Mengen von 0,02 bis 0,07 Gew.-%.

4. Konservierte Antacida-Zubereitungen nach Anspruch 1 enthaltend Hydrotalcit als Antacidum.

5. Konservierte Antacida-Zubereitungen nach Anspruch 1 in wäßrigen Suspensionen.

6. Konservierte Antacida-Zubereitungen nach Anspruch 1 in Form von wäßrigen Suspensionen enthaltend 1 - 40 Gew.-% Hydrotalcit und 0,01 bis 0,1 Gew.-% Zimtöl.

7. Verwendung von Zimtöl als alleiniges Konservierungsmittel in Antacida-Zubereitungen.

8. Verwendung von Zimtöl nach Anspruch 7 in wäßrigen Hydrotalcit-Suspensionen.

9. Verfahren zur Herstellung von konservierten Antacida-Zubereitungen enthaltend Zimtöl als einziges Konservierungsmittel, dadurch gekennzeichnet, daß man den Zubereitungen als Konservierungsmittel Zimtöl zusetzt.

10. Verfahren nach Anspruch 9, dadurch gekennzeichnet, däß man eine wäßrige Suspension, enthaltend 1 - 40 Gew.-% Hydrotalcit mit 0,01 bis 0,1 Gew.-% Zimtöl und 0,5 bis 5 Gew.-% Verdickungsmittel vermischt und anschließend homogenisiert.

**Patentansprüche für folgenden Vertragsstaat : ES**

1. Verfahren zur Herstellung von konservierten Antacida-Zubereitungen enthaltend Zimtöl als einziges Konservierungsmittel, dadurch gekennzeichnet, daß man den Zubereitungen als Konservierungsmittel Zimtöl zusetzt.

2. Verfahren nach Anspruch 1, dadurch gekennzeichnet, daß man eine wäßrige Suspension, enthaltend 1 -

EP 0 241 804 B1

40 Gew.-% Hydrotalcit mit 0,01 bis 0,1 Gew.-% Zimtöl und 0,5 bis 5 Gew.-% Verdickungsmittel vermischt und anschließend homogenisiert.

**Claims**

**Claims for the following Contracting States : AT, BE, CH, LI, DE, FR, GB, IT, NL**

1. Preserved antacid formulations, containing oil of cinnamon as sole preservative.

2. Preserved antacid formulations according to Claim 1 containing oil of cinnamon in amounts of 0.01 to 0.1% by weight.

3. Preserved antacid formulations according to Claim 1 containing oil of cinnamon in amounts of 0.02 to 0.07% by weight.

4. Preserved antacid formulations according to Claim 1 containing hydrotalcite as antacid.

5. Preserved antacid formulations according to Claim 1 in aqueous suspensions.

6. Preserved antacid formulations according to Claim 1 in the form of aqueous suspensions containing 1 - 40% by weight of hydrotalcite and 0.01 to 0.1% by weight of oil of cinnamon.

7. Use of oil of cinnamon as sole preservative in antacid formulations.

8. Use of oil of cinnamon according to Claim 7 in aqueous hydrotalcite suspensions.

9. Process for the preparation of preserved antacid formulations containing oil of cinnamon as sole preservative, characterised in that oil of cinnamon is added to the formulations as preservative.

10. Process according to Claim 9, characterised in that an aqueous suspension containing 1 - 40% by weight of hydrotalcite is mixed with 0.01 to 0.1% by weight of oil of cinnamon and 0.5 to 5% by weight of thickener and the mixture is then homogenised.

**Claims for the following Contracting State : ES**

1. Process for the preparation of preserved antacid formulations containing oil of cinnamon as sole preservative, characterised in that oil of cinnamon is added to the formulations as preservative.

2. Process according to Claim 1, characterised in that an aqueous suspension containing 1 - 40% by weight of hydrotalcite is mixed with 0.01 to 0.1% by weight of oil of cinnamon and 0.5 to 5% by weight of thickener and the mixture is then homogenised.

**Revendications**

**Revendications pour les Etats contractants suivants : AT, BE, CH, LI, DE, FR, GB, IT, NL**

1. Compositions anti-acides conservées, contenant de l'essence de cannelle en tant qu'unique agent conservateur.

2. Compositions anti-acides conservées selon la revendication 1, contenant l'essence de cannelle en quantité de 0,01 à 0,1 % en poids.

3. Compositions anti-acides conservées selon la revendication 1, contenant l'essence de cannelle en quantité de 0,02 à 0,07 % en poids.

4. Compositions anti-acides conservées selon la revendication 1, contenant de l'hydrotalcite en-tant qu'agent anti-acide.

5. Compositions anti-acides conservées selon la revendication 1, en suspension aqueuse.

9

**6.** Compositions anti-acides conservées selon la revendication 1, à l'état de suspensions aqueuses contenant 1 à 40 % en poids d'hydrotalcite et 0,01 à 0,1 % en poids d'essence de cannelle.

**7.** Utilisation de l'essence de cannelle en tant qu'unique agent conservateur dans des compositions anti-acides.

**8.** Utilisation de l'essence de cannelle selon la revendication 7, dans des suspensions aqueuses d'hydrotalcite.

**9.** Procédé de préparation de compositions anti-acides conservées, contenant de l'essence de cannelle en tant qu'unique agent conservateur, caractérisé en ce que l'on ajoute l'essence de cannelle en tant qu'agent conservateur aux compositions.

**10.** Procédé selon la revendication 9, caractérisé en ce que l'on forme par mélange une suspension aqueuse contenant 1 à 40 % en poids d'hydrotalcite avec 0,01 à 0,1 % en poids d'essence de cannelle et 0,5 à 5 % en poids d'un agent épaississant puis on homogénéise.

**Revendications pour l'Etat contractant suivant : ES**

**1.** Procédé de préparation de compositions anti-acides conservées, contenant de l'essence de cannelle en tant qu'unique agent conservateur, caractérisé en ce que l'on ajoute aux compositions de l'essence de cannelle en tant qu'agent conservateur.

**2.** Procédé selon la revendication 1, caractérisé en ce que l'on mélange une suspension aqueuse contenant 1 à 40 % en poids d'hydrotalcite avec 0,01 à 0,1 % en poids d'essence de cannelle et 0,5 à 5 % en poids d'un agent épaississant puis on homogénéise.